# FASCICULE DE BREVET EUROPEEN

(11) **EP 3 801 013 B1**
(45) Date de publication et mention de la délivrance du brevet: **27.09.2023**
(21) Numéro de dépôt: 19742836.0
(22) Date de dépôt: 31.05.2019
(51) Int. Cl.: A01K 67/033

(54) **MILIEU DE PONTE POUR INSECTES COMPORTANT UN SUBSTRAT SOLIDE**
EILEGEMITTEL FÜR INSEKTEN MIT EINEM FESTEN SUBSTRAT
EGG-LAYING MEDIUM FOR INSECTS, COMPRISING A SOLID SUBSTRATE

(30) Priorité: 01.06.2018 FR 1854800
(43) Date de publication de la demande: 14.04.2021
(73) Titulaire: Ynsect, 91058 Evry-Courcouronnes Cedex (FR)
(72) Inventeur: MATHIEU, Marianne, 77140 SAINT PIERRE LES NEMOURS (FR); ESCALANTE NOGUERA, Pedro, 91260 JUVISY SUR ORGE (FR); BERRO, Fabrice, 75001 PARIS (FR); DU JONCHAY, Thibault, 60710 CHEVRIÈRES (FR); BEREZINA, Nathalie, 1772 Järfälla (SE)
(74) Mandataire: Santarelli
(86) Numéro de dépôt international: PCT/FR2019/051285
(87) Numéro de publication internationale: WO 2019/229397

(56) Documents cités:
- WO-A2-2014/046529
- FR-A1- 2 835 701
- US-A1- 2009 000 553
- Fao: "texture du sol", , 31 juillet 2013 (2013-07-31), XP055551113, Extrait de l'Internet: URL:http://blog.ac-versailles.fr/formation capa/public/le_sol/LE_SOL__2_.pdf [extrait le 2019-02-04]
- HOUSE H L: "AN ARTIFICIAL HOST: ENCAPSULATED SYNTHETIC MEDIUM FOR IN VITRO OVIPOSITION AND REARING THE ENDOPARASITOID ITOPLECTIS CONQUISITOR (HYMENOPTERA: ICHNEUMONIDAE)", CANADIAN ENTOMOLOGIST, OTTAWA, CA, vol. 110, no. 3, 1 mars 1978 (1978-03-01), pages 331-333, XP001027170, ISSN: 0008-347X

## Description

La présente invention concerne l'élevage d'insectes et plus particulièrement l'élevage des coléoptères et/ou des lépidoptères. Elle concerne plus particulièrement un milieu de ponte et un bac de ponte et leurs utilisations, notamment dans un procédé de collecte d'oeufs d'insectes.

L'élevage d'insectes connaît un essor important ces dernières années. La production d'insectes présente de nombreux intérêts, que ce soit pour l'agro-industrie, les insectes constituant une source intéressante de protéines, ou dans d'autres domaines industriels, les insectes étant également une source de chitine, transformable en chitosan, dont les applications sont nombreuses : cosmétique, médicale et pharmaceutique, diététique et alimentaire, traitement de l'eau, etc.

L'élevage d'insectes à l'échelle industrielle suppose de pouvoir faire se reproduire de manière efficace les insectes.

Le plus souvent, en élevage, les femelles insectes pondent des oeufs dans leur milieu nutritif. Ces oeufs, souvent très petits, éclosent quelques semaines après la ponte. Or, il arrive que les larves qui viennent d'éclore dévorent les oeufs non encore éclos. D'où la nécessité de bien séparer les oeufs de la population larvaire afin de maintenir une production élevée.

Mais il existe donc un besoin pour un procédé de collecte d'oeufs efficace à l'échelle industrielle. Le document FR-A-2835701 vise à fournir un milieu encapsulé pour former un objet de type hôte (« host-like object ») dans lequel un insecte parasite pond, puis dans lequel l'éclosion a lieu.

KR20130046658 est relatif à une méthode pour recueillir les oeufs d'un insecte *Tenebrio molitor* (ou *T. molitor*) comprenant un récipient et un filet de filtration amovible, la méthode comprenant notamment les étapes suivantes : introduire dans un récipient de la farine de céréales, introduire les individus au stade adulte dans le filet de filtration amovible, inciter les femelles à pondre des oeufs de manière à ce que ceux-ci soient collés à la paroi du récipient, et récupérer les oeufs d'une part et, grâce au filet de filtration amovible, les femelles d'autre part.

Cependant, ce document ne décrit pas clairement comment les femelles sont incitées à pondre les oeufs de manière à ce que ceux-ci soient collés à la paroi du récipient. De plus, une telle méthode n'est pas adaptée à une échelle industrielle, impliquant productivité, notamment surfacique, élevée.

La présente invention vise à proposer un procédé de collecte d'œufs d'insectes permettant de pallier les inconvénients ci-avant. Par « oeufs d'insectes », on vise plus particulièrement des oeufs d'insectes isolés, c'est-à-dire n'étant pas sous forme d'amas appelés oothèques.

Le travail des inventeurs leur a permis d'élaborer ce procédé de collecte, qui nécessite l'utilisation d'un milieu de ponte spécifique.

L'invention concerne donc un milieu de ponte pour insectes comportant :
∘ au moins 80% en poids d'un substrat solide destiné à être consommé par les insectes sous forme de particules, au moins 85% en poids desdites particules ayant une taille de particules inférieure à 0,5 mm, ledit substrat solide ayant une humidité comprise entre 0 et 15%,
le pourcentage en poids étant donné sur le poids total de milieu de ponte pour insectes.

On notera que, dans le cadre de la présente demande, et sauf stipulation contraire, les gammes de valeurs indiquées s'entendent bornes incluses.

Par « substrat solide », on vise un substrat solide ou un mélange de substrats solides, destiné à être consommé par les insectes.

De préférence, le milieu de ponte pour insectes comporte :
∘ de 90% à 98% en poids d'un substrat solide sous forme de particules, au moins 85% en poids desdites particules ayant une taille de particules inférieure à 0,5 mm, et ledit substrat solide ayant une humidité comprise entre 0 et 15%,
le pourcentage en poids étant donné sur le poids total de milieu de ponte pour insectes.

La taille de particules est une caractéristique bien connue de l'homme du métier, qui permet de caractériser des compositions telles que, par exemple, des poudres, des moutures.

A titre d'exemple, le substrat solide peut être en effet sous forme d'une poudre, d'une mouture.

La granulométrie est l'étude de la distribution de la taille des particules d'une composition. Les techniques d'analyse granulométrique sont bien connues de l'homme du métier. A titre d'exemple, on pourra se référer à la publication suivant : « La granulométrie de l'aliment : principe, mesure et obtention ; INRA Prod. Anim., 2000, 13 (2), 81-97 ». Et à la publication: AN ARTIFICIAL HOST: ENCAPSULATED SYNTHETIC MEDIUM FOR IN VITRO OVIPOSITION AND REARING THE ENDOPARASITOID ITOPLECTIS CONQUISITOR (HYMENOPTERA: ICHNEUMONIDAE), CANADIAN ENTOMOLOGIST, vol 110, nr 3, pages 331-333.

Par « particules ayant une taille inférieure à Y », on entend des particules qui passent à travers un tamis ayant un vide de maille de Y.

Préférentiellement, au moins 90% en poids des particules du substrat solide a une taille inférieure à 0,5 mm.

Préférentiellement, le substrat solide a une humidité comprise entre 0 et 10%.

De préférence, le substrat solide est un produit ou co-produit solide issu de la transformation des céréales, des oléogineux, des oléoprotéagineux et/ou des protéagineux.

Un coproduit est une matière inévitable créée au cours d'un processus de fabrication d'un produit d'intérêt.

Plus particulièrement, le substrat solide est avantageusement un produit ou coproduit issu de la transformation du blé (blé tendre, blé dur), du maïs, de l'orge, du riz, du triticale, de l'avoine, du sorgho, du seigle, de l'épeautre, du millet, du quinoa, du sarrasin, du colza, du tournesol, du lin, du soja et/ou du pois.

De préférence, le substrat solide est un produit ou coproduit issu de la transformation du blé, plus préférentiellement, le substrat est du son de blé et/ou du remoulage de blé.

Alternativement, des drêches sèches de distillerie avec soluble (« Dried Distilled Grains with Solubles ») peuvent être utilisées.

Comme indiqué ci-avant, le substrat solide doit avoir une taille de particules inférieure à 0,5 mm.

Cette taille de particules permet une séparation aisée et propre des oeufs d'insectes des autres constituants du milieu de ponte.

Toutefois, si le produit ou co-produit solide issu de la transformation des céréales, des oléogineux, des oléoprotéagineux et/ou des protéagineux a une taille de particules supérieure à 0,5 mm, celui-ci pourra subir une étape de broyage, de sorte à obtenir un substrat solide ayant une taille de particules inférieure à 0,5 mm. C'est le cas par exemple du son de blé, 75% en poids des particules de son de blé ayant une taille de particule supérieure à 0,8 et inférieure à 1,4 mm.

Cette étape de broyage peut être réalisée à l'aide de tout type de broyeur adapté. Ces broyeurs sont bien connus de l'homme du métier.

Le substrat solide (ou le mélange de substrats solides) présente une humidité comprise entre 0 et 15%, préférentiellement entre 0 et 10%. De ce fait, un apport d'eau complémentaire est nécessaire. Cet apport d'eau complémentaire peut par exemple être effectué via l'ajout d'eau sous forme pulvérisée, l'introduction de végétaux aqueux, tel que par exemple, des végétaux ayant au moins 60% d'humidité (carottes, pommes de terre, etc.) et/ou via l'introduction d'un gel aqueux et optionnellement nutritif.

Lorsqu'un gel aqueux et optionnellement nutritif est utilisé pour effectuer l'apport d'eau, celui-ci est introduit à une quantité d'au moins 2% en poids, sur le poids total du milieu de ponte pour insectes.

L'invention concerne donc plus particulièrement un milieu de ponte pour insectes comportant :
∘ au moins 80% en poids d'un substrat solide sous forme de particules, au moins 85% en poids desdites particules ayant une taille de particules inférieure à 0,5 mm, ledit substrat solide ayant une humidité comprise entre 0 et 15%, et
∘ au moins 2% en poids d'un gel aqueux et optionnellement nutritif, les pourcentages en poids étant donnés sur le poids total de milieu de ponte pour insectes.

Préférentiellement, le milieu de ponte pour insecte comporte :
∘ de 95 à 97% d'un substrat solide sous forme de particules, au moins 85% en poids desdites particules ayant une taille de particules inférieure à 0,5 mm, ledit substrat solide ayant une humidité comprise entre 0 et 15%, et
∘ de 3% à 5% en poids d'un gel aqueux et optionnellement nutritif, les pourcentages en poids étant donnés sur le poids total de milieu de ponte pour insectes.

Avantageusement, le gel aqueux et optionnellement nutritif comporte :
∘ au moins 90% poids d'une solution aqueuse,
∘ de 0,3 à 2% en poids d'un agent gélifiant, et
∘ de 0,1 à 5% en poids d'un agent conservateur,
les pourcentages en poids étant exprimés sur le poids total du gel.

De préférence, le gel aqueux et optionnellement nutritif a une teneur en eau supérieure à 50%, préférentiellement supérieure à 70%, plus préférentiellement encore supérieure à 90% en poids sur le poids total de gel.

Selon un premier mode de réalisation du gel aqueux et optionnellement nutritif, la solution aqueuse est constituée d'eau.

Selon un second mode de réalisation du gel aqueux et optionnellement nutritif, le gel est également nutritif et la solution aqueuse peut comporter, outre de l'eau, un co-produit liquide de l'agro-industrie. De préférence, l'agro-industrie est choisie parmi les industries de l'amidonnerie, de la féculerie, de la malterie, de production de bioéthanol, du sucre, de la fermentation, de la brasserie, de la distillation et l'industrie laitière. De préférence, le coproduit liquide de l'agro-industrie est choisi parmi la liste constituée par les solubles de céréales, les solubles de maïs, les solubles de blé, les solubles de pois, les solubles de manioc, les solubles de betterave à sucre, les solubles de canne à sucre, les solubles de distillerie de céréales, les solubles de distillerie de blé, les solubles de distillerie de maïs, les solubles de distillerie de pois, les solubles de distillerie du manioc, les vinasses, les mélasses, les crèmes de levures, les lactosérums et leurs dérivés concentrés notamment le perméat, ou leurs mélanges. Plus préférentiellement, le coproduit liquide de l'agro-industrie est choisi parmi un soluble de distillerie ou un mélange d'un soluble de distillerie avec un autre coproduit liquide.

Avantageusement, l'agent gélifiant choisi parmi le groupe constitué par la gomme de xanthane, la gomme de caroube, la gomme de guar, ou leur mélange. De préférence, l'agent gélifiant est un mélange de gomme de xanthane et de gomme de caroube ou de gomme de xanthane et de gomme de guar.

Le gel aqueux et optionnellement nutritif peut également comporter des levures, des vitamines et/ou des probiotiques.

Avantageusement, le gel aqueux et optionnellement nutritif a une force de gel d'au moins 20 g/cm², préférentiellement, 30 g/cm², plus préférentiellement 50 g/cm².

Cette force de gel permet l'obtention d'un gel solide et de structure peu visqueuse, qui ne sera pas asséché par la présence de particules fines susceptible d'adhérer au gel.

De préférence, le substrat solide a une taille de particules comprise entre 0,3 et 0,5 mm, c'est-à-dire qu'au moins 50% des particules a une taille supérieure à 0,3 et inférieure à 0,5 mm.

Une taille de particules de substrat comprise entre 0,3 et 0,5 mm a notamment pour avantage d'éviter le dessèchement du gel aqueux.

De par le choix des différents paramètres exposés ci-avant, le milieu de ponte pour insectes selon l'invention permet notamment :
- La séparation aisée et propre des oeufs d'insectes des autres constituants du milieu de ponte : adultes, restes d'adultes morts, excréments (frass), substrats et éventuellement du gel. Ceci permet notamment d'augmenter les densités dans la production, en obtenant une meilleure productivité par unité de surface ;
- De résoudre les problèmes de développement des parasites opportunistes (mites, mouches, etc.) qui peuvent coloniser les substrats nutritifs consommés de manière insuffisante par les adultes et petites larves. La présence de substrats non consommés pendant une période étendue est principalement due aux difficultés de concentrer les insectes du stade de l'œuf jusqu'à la phase des larves de 20 mg. Cela est également effectué par un contrôle de l'humidité ;
- D'augmenter les performances de reproduction. Cette augmentation peut s'expliquer notamment par le choix du milieu de ponte, qui favorise notamment une diminution de la consommation accidentelle des oeufs par les adultes. Or, usuellement, dans les conditions densifiées, la probabilité d'occurrence de tels évènements est plus élevée. Le choix du milieu de ponte permet une collecte facilitée des oeufs, collecte dont la fréquence peut être augmentée et qui limite ainsi ce phénomène ;
- Tout en maintenant une productivité élevée.

Le milieu de ponte selon l'invention est avantageusement disposé sur le fond d'un contenant afin de former un bac de ponte.

L'invention concerne également un bac de ponte comportant un contenant et, sur un fond dudit contenant :
∘ de 0,12 à 7,5 g/cm² d'un substrat solide destiné à être consommé par les insectes sous forme de particules, au moins 85% en poids desdites particules ayant une taille de particules inférieure à 0,5 mm, ledit substrat solide ayant une humidité comprise entre 0 et 15%.

Les quantités surfaciques sont exprimées par rapport à la surface du fond du contenant. Un tel bac de ponte est adapté aux insectes.

De préférence, le bac de ponte selon l'invention comporte en outre sur le fond dudit contenant :
∘ de 0,006 à 0,325 g/cm² ou 0,0016 à 0,095 g/cm²/j d'un gel aqueux et optionnellement nutritif.

On notera que les quantités en g/cm²/j dépendent du temps de séjour en jour (j) des insectes. Typiquement, la quantité en g/cm²/j indiquée ci-avant correspond à un séjour de 7j des insectes, séjour au cours duquel la teneur en gel sera renouvelée une fois.

De préférence, la surface du fond du contenant du bac de ponte comporte :
o de 0,17 à 6,95 g/cm² d'un substrat solide sous forme de particules, au moins 85% en poids desdites particules ayant une taille de particules inférieure à 0,5 mm, ledit substrat solide ayant une humidité comprise entre 0 et 15%, et
∘ de 0,0074 à 0,275 g/cm² ou 0,0022 à 0,08 g/cm²/j d'un gel aqueux et optionnellement nutritif.

De préférence, notamment, dans le bac de ponte :
∘ le substrat solide sous forme de particules est présent à une teneur de 90% à 98% en poids, au moins 85% en poids desdites particules ayant une taille de particules inférieure à 0,5 mm ;
∘ au moins 90% en poids des particules du substrat solide a une taille inférieure à 0,5 mm ;
∘ le substrat solide a une humidité comprise entre 0 et 10% ;
∘ le substrat solide est un produit ou co-produit solide issu de la transformation des céréales et/ou des oléogineux/oléoprotéagineux pouvant présenter les caractéristiques particulières, avantageuses et préférées telles qu'indiqués pour le milieu de ponte ci-avant ;
∘ le gel aqueux et optionnellement nutritif comporte :
   ∘ au moins 90% en poids d'une solution aqueuse,
   ∘ de 0,3 à 2% en poids d'un agent gélifiant, et
   ∘ de 0,1 à 5% en poids d'un agent conservateur,
   les pourcentages en poids étant exprimés sur le poids total du gel ; ledit gel aqueux et optionnellement nutritif pouvant présenter les caractéristiques particulières, avantageuses et préférées telles qu'indiqués pour le milieu de ponte ci-avant ; en particulier, le gel aqueux et optionnellement nutritif a une teneur en eau supérieure à 50%, préférentiellement supérieure à 70%, plus préférentiellement encore supérieure à 90% en poids sur le poids total de gel.

L'invention se rapporte également à l'utilisation d'un milieu de ponte selon l'invention, d'un bac de ponte selon l'invention pour l'élevage de coléoptères et/ou lépidoptères.

Par coléoptères et/ou lépidoptères, on vise plus particulièrement les coléoptères et lépidoptères appartenant aux familles des Tenebrionidae, Melolonthidae, Dermestidae, Coccinellidae, Cerambycidae, Carabidae, Buprestidae, Cetoniidae, Dryophthoridae, Silvanidae, Trogoderma, Laemophloeidae, Trogossitidae, Pyralidae ou leurs mélanges.

Plus préférentiellement, il s'agit des coléoptères et/ou lépidoptères suivants : *Tenebrio molitor, Tenebrio obscurus, Tribolium castaneum, Tribolium confusum, Dermestes ater, Dermestes magister, Alphitobius diaperinus, Zophobas morio, Rhynchophorus ferrugineus, Oryzaephilus surinamensis, Cryptolestes ferrugineus, Trogoderma granarium, Gnathocerus cornutus, Tenebroides mauritanicus et Ephestia kuehniella.*

Plus préférentiellement, le milieu de ponte selon l'invention et le bac de ponte selon l'invention sont mis en oeuvre dans l'élevage de coléoptères, en particulier des familles des Tenebrionidae, Melolonthidae, Dermestidae, Coccinellidae, Cerambycidae, Carabidae, Buprestidae, Cetoniidae et Dryophthoridae.

Plus préférentiellement, il s'agit des coléoptères *Tenebrio molitor, Tenebrio obscurus, Tribolium castaneum, Alphitobius diaperinus, Zophobas morio, Rhynchophorus ferrugineus,* ou leur mélange, et plus particulièrement dans l'élevage de *Tenebrio molitor.*

L'invention concerne enfin un procédé d'obtention d'oeufs d'insectes comportant les étapes de :
- obtention d'un bac de ponte par la fourniture d'un contenant et le remplissage dudit contenant avec :
   ∘ un substrat solide destiné à être consommé par les insectes sous forme de particules, au moins 85% en poids desdites particules ayant une taille de particules inférieure à 0,5 mm, ledit substrat solide ayant une humidité comprise entre 0 et 15%,
   pour obtenir un bac de ponte,
- introduction d'insectes adultes dans le bac de ponte, et
une étape subséquente de collecte des oeufs d'insectes.

Dans le procédé d'obtention d'oeufs d'insectes selon l'invention, l'étape de remplissage du contenant par le substrat solide est effectuée par apport de 0,12 à 7,5 g/cm² dudit substrat solide dans le contenant.

Préférentiellement, l'étape de remplissage du contenant par le substrat solide est effectuée par un apport de 0,17 à 6,95 g/cm² de substrat solide.

Avantageusement, l'apport de substrat solide dans le contenant s'effectue sur une hauteur de 1 à 5 cm, préférentiellement sur une hauteur de 2 à 4 cm.

Préférentiellement, dans le procédé d'obtention d'oeufs d'insectes selon l'invention, l'étape de remplissage du contenant comporte l'introduction d'un gel aqueux et optionnellement nutritif à un apport de 0,0016 à 0,095 g/cm²/j, plus préférentiellement à un apport de 0,0022 à 0,08 g/cm²/j

La quantité de gel aqueux et optionnellement nutritif en g/cm²/j dépend du temps de séjour en jour (j) des insectes dans le bac de ponte. Ce temps de séjour correspond au nombre de jours écoulé depuis l'introduction des insectes dans le bac de ponte et l'étape de collecte des oeufs.

Pour un temps de séjour de 3,5 jours, la quantité de gel aqueux et optionnellement nutritif est de 0,006 à 0,325 g/cm², préférentiellement, de 0,0074 à 0,275 g/cm².

Avantageusement, dans le procédé d'obtention d'oeufs d'insectes selon l'invention, l'étape d'introduction des insectes adultes dans le bac de ponte est effectuée à une densité surfacique comprise entre 0,01 et 1,0 g/cm², préférentiellement à une densité surfacique comprise entre 0,02 à 0,75 g/cm².

Préférentiellement, le procédé d'obtention d'oeufs d'insectes selon l'invention comporte les étapes suivantes :
- obtention d'un bac de ponte par la fourniture d'un contenant et le remplissage d'un fond dudit contenant avec :
   ∘ 0,17 à 6,95 g/cm² d'un substrat solide sous forme de particules, au moins 85% en poids desdites particules ayant une taille de particules inférieure à 0,5 mm, ledit substrat solide ayant une humidité comprise entre 0 et 15%,
   ∘ 0,0022 à 0,08 g/cm²/j d'un gel aqueux et optionnellement nutritif,
- introduction d'insectes adultes dans le bac de ponte, et une étape subséquente de collecte des oeufs d'insectes.

Alternativement, le procédé d'obtention d'oeufs d'insectes selon l'invention comporte les étapes suivantes :
∘ obtention d'un bac de ponte par la fourniture d'un contenant et le remplissage d'un fond dudit contenant avec :
   ∘ 0,17 à 6,95 g/cm² d'un substrat solide sous forme de particules, au moins 85% en poids desdites particules ayant une taille de particules inférieure à 0,5 mm, ledit substrat solide ayant une humidité comprise entre 0 et 1 5%,
   ∘ 0,0074 à 0,275 g/cm²d'un gel aqueux et optionnellement nutritif, ∘ introduction d'insectes adultes dans le bac de ponte, et
une étape subséquente de collecte des oeufs d'insectes.

Un tel procédé est adapté pour un séjour de 7j des insectes dans le bac de ponte, séjour au cours duquel la teneur en gel sera renouvelée une fois, par exemple au cours du jour 3, le jour 1 étant le jour d'introduction des insectes adultes dans le bac de ponte.

Selon un mode de réalisation particulièrement avantageux, l'étape subséquente de collecte des oeufs du procédé d'obtention d'oeufs d'insectes selon l'invention est effectuée au moyen d'une étape de tri automatisé.

L'étape de tri automatisé permet une séparation aisée des différents éléments contenus dans le bac de ponte.

Cette étape de tri automatisé peut être effectuée aux moyens de dispositifs tels que des tamiseurs à nutation oscillatoire ou des tamiseurs vibratoires linéaires.

Ces dispositifs permettent de séparer aisément différentes fractions, classées ci-après par taille croissante :
∘ La fraction de substrat solide,
∘ La fraction d'excrétions d'insectes adultes,
∘ La fraction d'oeufs d'insectes,
∘ La fraction des restes d'insectes adultes morts,
∘ La fraction des insectes adultes.

A l'issue de l'étape de tri automatisé, la récupération de la fraction d'oeufs d'insectes permet la collecte des oeufs.

La fraction d'oeufs pouvant comporter des impuretés, il est possible d'effectuer une étape supplémentaire de séparation afin d'obtenir des oeufs purs et propres. Dans ce cas, la fraction d'oeufs subit une séparation densimétrique avec une vitesse de ventilation adaptée à la quantité d'oeufs pour permettre l'obtention d'oeufs purs et propres.

Alternativement, il est possible d'utiliser la fraction d'oeufs telle quelle.

Par ailleurs, le tri automatisé permet également de séparer et collecter les insectes adultes. Les adultes vivants peuvent ensuite être séparés des adultes morts à l'aide d'une colonne densimétrique. Une fois séparés, les insectes adultes vivants peuvent être réutilisés pour peupler un nouveau bac de ponte selon l'invention.

Préférentiellement, l'étape de collecte du procédé d'obtention d'oeufs d'insectes selon l'invention est effectuée tous les 2 à 3 jours.

Une récolte tous les 2 à 3 jours permet d'augmenter les performances de ponte d'au moins 20%.

De préférence, notamment, dans le procédé d'obtention d'oeufs d'insectes selon l'invention :
∘ le substrat solide sous forme de particules est présent à une teneur de 90% à 98% en poids, au moins 85% en poids desdites particules ayant une taille de particules inférieure à 0,5 mm ;
∘ au moins 90% en poids des particules du substrat solide a une taille inférieure à 0,5 mm ;
∘ le substrat solide a une humidité comprise entre 0 et 10% ;
∘ le substrat solide est un produit ou co-produit solide issu de la transformation des céréales et/ou des oléogineux/oléoprotéagineux pouvant présenter les caractéristiques particulières, avantageuses et préférées telles qu'indiqués pour le milieu de ponte ci-avant ;
∘ le gel aqueux et optionnellement nutritif comporte :
   ∘ au moins 90% en poids d'une solution aqueuse,
   ∘ de 0,3 à 2% en poids d'un agent gélifiant, et
   ∘ de 0,1 à 5% en poids d'un agent conservateur,
   les pourcentages en poids étant exprimés sur le poids total du gel ; ledit gel aqueux et optionnellement nutritif pouvant présenter les caractéristiques particulières, avantageuses et préférées telles qu'indiqués pour le milieu de ponte ci-avant ; en particulier, le gel aqueux et optionnellement nutritif a une teneur en eau supérieure à 50%, préférentiellement supérieure à 70%, plus préférentiellement encore supérieure à 90% en poids sur le poids total de gel.

Le procédé d'obtention d'oeufs d'insectes selon l'invention est particulièrement adapté pour l'élevage de coléoptères et/ou lépidoptères. Les coléoptères et/ou lépidoptères préférés sont tels qu'indiqués ci-avant, et plus préférentiellement, le procédé d'obtention d'oeufs d'insectes selon l'invention est particulièrement adapté pour l'élevage de *T. molitor.*

D'autres caractéristiques et avantages de l'invention apparaîtront dans les exemples qui suivent, donnés à titre illustratif, avec référence aux figures, qui représentent respectivement :
- La Figure 1 est un schéma d'un bac de ponte selon l'invention,
- La Figure 2 est un schéma représentant la séparation des différentes fractions lors de l'étape automatisée de tri et de collecte des oeufs d'insectes,
- La Figure 3 est composées de deux photographies représentant deux fractions d'oeufs issues d'un tri d'un bac de ponte : sur la figure de gauche, la taille des particules de substrat solide n'a pas été sélectionnée tandis que sur la figure de droite, la fraction d'oeufs a été triée selon la méthode selon l'invention, le substrat solide ayant une granulométrie adaptée au tri des oeufs (particules inférieures à 0,5mm), et
- La Figure 4 est un schéma de mise en oeuvre « en continu » du procédé d'obtention d'oeufs d'insectes.

### Exemple I : Préparation d'un milieu de ponte et d'un bac de ponte

Le milieu de ponte est préparé à l'aide des trois composants suivants :
- Substrat solide : du remoulage blanc de blé, ayant une humidité de 7%, dont la granulométrie est la suivante :
   ▪ 65% en poids des particules ont une taille comprise entre 0,30 et 0,50 mm,
   ▪ 30% en poids des particules ont une taille inférieure à 0,30 mm, et
   ▪ 5% en poids des particules ont une taille comprise entre 0,50 et 0,80 mm.
- Gel : un gel aqueux est préparé à partir de 98,7% en poids d'eau, 1% en poids d'agent gélifiant (Flanogen) et 0,3% en poids de sorbate de potassium.

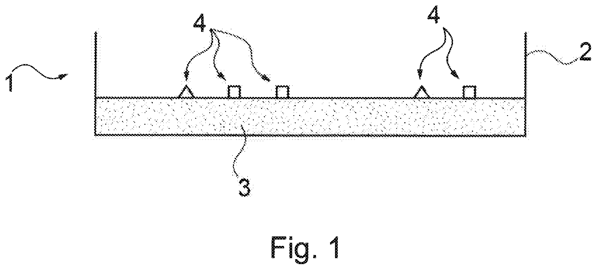

La Figure 1 représente un bac de ponte 1.

Dans un contenant 2 en plastique, on place :
- Un substrat solide 3, à savoir 2000-4000 g de remoulage blanc de blé (1-2 g/cm²) ;
- Un gel aqueux et optionnellement nutritif 4, à savoir 75-112,5 g de gel aqueux (0,037-0,055 g/cm²).

De préférence, les composants sont introduits dans l'ordre indiqués ci-avant.

On obtient ainsi un bac de ponte.

### Exemple II : Procédé d'obtention d'oeufs d'insectes

### 1. Matériel

- De jeunes adultes *Tenebrio molitor* (âgés d'1 semaine)
- Des bacs de ponte de l'Exemple I
- Une salle d'élevage à température et humidité contrôlées
- Un tamiseur à nutation oscillatoire (« tumbler screening machine », Allgaier) ou un tamiseur vibratoire linéaire (« linear screening machine », Mogensen)

### 2. Méthodes

Tri des adultes : Pour peupler les bacs de ponte, une étape de tri à partir de ténébrions au stade nymphal peut être nécessaire. Les nymphes devenant adultes avec le temps, les adultes sont alors séparés des nymphes. Le tri entre les nymphes et les adultes est réalisé sur une période de temps qui ne dépasse pas 7 jours. Cette étape de tri permet ainsi d'obtenir une population adulte homogène (± 7 jours de différence d'âge au sein de la population) au sein du bac de ponte.

Création et peuplement du bac de ponte : Le bac de ponte est créé comme indiqué à l'Exemple I. Les adultes peuvent être soit issus de l'étape de tri ci-avant, soit issus d'un ancien bac de ponte. En effet, la population d'adultes pour la ponte est gardée pendant plusieurs semaines, par exemple 8 semaines tandis que les séjours des adultes dans un bac de ponte peuvent durer de 2 à 14 jours. Dès lors, à l'issue d'un séjour, les adultes sont à nouveau triés, notamment pour retirer les adultes morts et garder les adultes vivants, puis ces derniers sont placés à nouveau à la densité optimale d'adultes dans un bac de ponte.
- Ténébrions adultes : 0,02-0,75 g/cm²

Une fois les bacs de ponte peuplés d'adultes, ils sont conservés dans une salle d'élevage ayant entre 50% et 90% d'humidité relative. Il peut être utile de réalimenter les bacs de ponte en gel aqueux. Typiquement, on apporte une quantité de gel aqueux (0,0074-0,275 g/cm²) 2 fois par semaine (soit un apport initial et un apport subséquent, effectué 3,5 jours après l'apport initial). Les quantités de matières sont calculées selon la surface du bac d'élevage.

Tri/Collecte des oeufs : La fréquence de collecte des oeufs peut être adaptée entre 2 à 7 jours. Dans le présent Exemple, la collecte des oeufs a été effectuée au bout d'une période de 7 jours. Le jour 7, le bac de ponte est récupéré dans la salle d'élevage et son contenu est déversé dans un tamiseur. Le tamiseur possède un ensemble de tamis qui permet la séparation en fonction de leur taille, des différentes fractions du contenu du bac de ponte.

L'étape de tri et de collecte des oeufs a été testée avec deux types de machines différentes, un tamiseur vibratoire linéaire et un tamiseur à nutation oscillatoire. Les deux machines ont donné de bons résultats lors de l'étape de collecte.

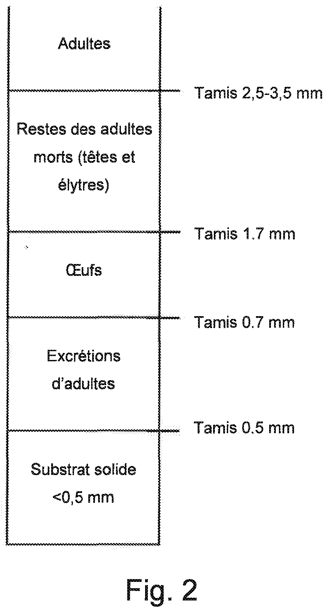

Sur la Figure 2, est décrite la séparation des différentes fractions en fonctions de leur taille :
- Fraction des insectes adultes : cette fraction correspond aux particules ne passant pas à travers (ou retenues par) un tamis ayant un vide de maille d'au plus 2,5 mm. Cette fraction comporte les adultes vivants et morts. Au cours d'une semaine de ponte d'oeufs dans le substrat solide (remoulage de blé), une mortalité d'environ 10% des individus est constatée. Les adultes vivants sont ensuite séparés des adultes morts à l'aide d'une séparation densimétrique. Les adultes vivants sont à nouveau déposés dans des bacs de ponte.
- Fraction des restes d'adultes morts : cette fraction correspond aux particules passant à travers un tamis ayant un vide de maille d'au plus 2,5 mm et retenues par un tamis ayant un vide de maille de 1,7 mm. Cette fraction comporte des parties d'adultes morts (têtes, pattes, etc.).
- Fraction d'oeufs d'insectes : cette fraction correspond aux particules passant à travers un tamis ayant un vide de maille de 1,7 mm et retenues par un tamis ayant un vide de maille de 0,7 mm. Cette fraction comporte les oeufs. La quantité d'oeufs obtenus dépendra des conditions du bac de ponte (densité de la population, substrats, gel). Dans les conditions de cet Exemple, une moyenne de 25,9 ± 5,68 œufs/cm² ou 0,0186 ± 0,0046 g d'œufs/cm² est obtenue. Les oeufs obtenus après l'étape de tamisage sont mélangés avec des impuretés : particules de substrat solide, quelques excrétions d'adultes et des restes d'adultes morts. En effet, généralement, après le tri, la fraction d'oeufs d'insectes comporte encore entre 50 et 60% en poids de particules, excrétions et restes (gros déchets).
- Fraction excrétions d'insectes adultes : cette fraction correspond aux particules passant à travers un tamis ayant un vide de maille de 0,7 mm et retenues par un tamis ayant un vide de maille de 0,5 mm. Cette fraction comporte les excrétions d'adultes.
- Fraction substrat solide : cette fraction correspond aux particules passant à travers un tamis ayant un vide de maille de 0,5 mm. Ce substrat solide peut alors être potentiellement réutilisé.
- Enfin, lorsque l'apport de gel aqueux est effectué dans les quantités indiquées ci-avant, celui-ci est généralement entièrement consommé par les insectes. Si le gel aqueux n'est pas consommé, un tamis de maille de 5 mm peut être utilisé, afin de récupérer les morceaux de gel séché.

La récupération de la fraction d'oeufs d'insectes permet la collecte des oeufs. Comme indiqué ci-avant, cette fraction encore entre 50 et 60% en poids de particules, excrétions et restes (gros déchets). Dès lors, elle peut être utilisée telle quelle, ou après une étape supplémentaire de séparation afin d'obtenir une fraction d'oeufs purs et propres. Dans ce cas la fraction d'œufs d'insectes subit une séparation densimétrique, telle qu'une séparation sur colonne densimétrique, avec une vitesse de ventilation adaptée à la quantité d'oeufs pour permettre l'obtention d'oeufs purs et propres. La fraction d'oeufs purs et propres comporte alors de 65 à 75% en poids d'oeufs, une part importante des 25 à 35% en poids restant étant de fines particules de substrat solides.

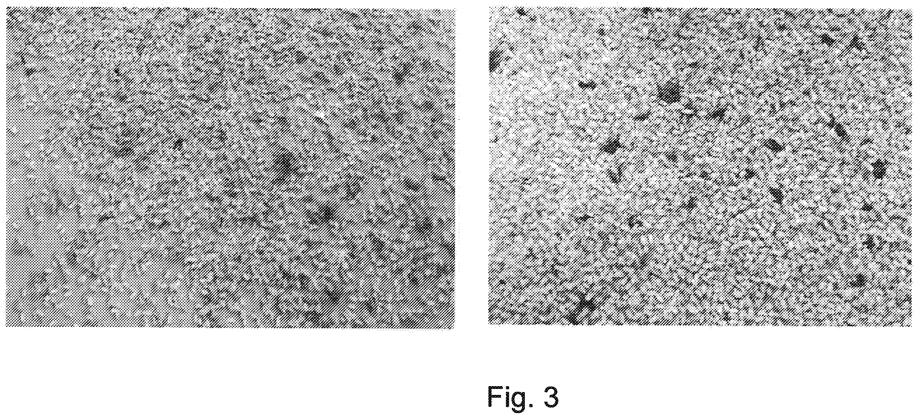

Sur la Figure 3, sont représentées deux fractions d'oeufs issues d'un tri d'un bac de ponte. Sur la figure de gauche, la taille des particules de substrat solide n'a pas été sélectionnée tandis que sur la figure de droite, la fraction d'oeufs a été triée selon la méthode selon l'invention, le substrat solide ayant une granulométrie adaptée au tri des oeufs particules inférieures à 0,5mm, ce qui conduit à une fraction d'oeufs de pureté supérieure.

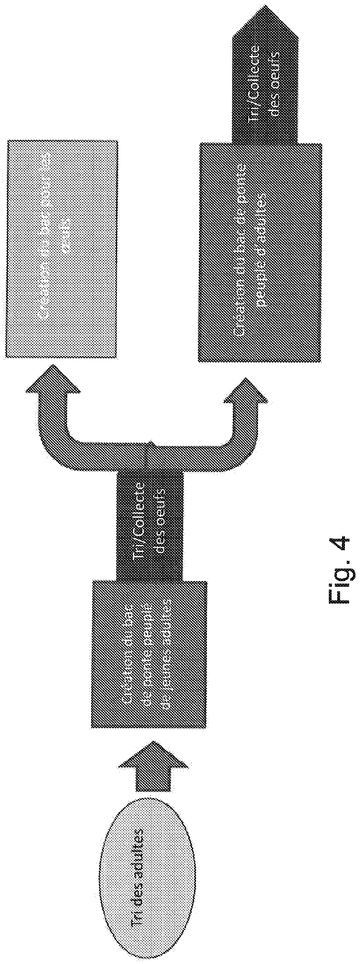

Sur la Figure 4, est représenté un schéma de mise en oeuvre « en continu » du procédé d'obtention d'oeufs d'insectes. Cette Figure est plus amplement détaillée ci-après :
Tri des adultes : voir la description ci-avant de cette étape.
Bac de pontes peuplé de jeunes adultes : comme indiqué ci-avant un bac de ponte est créé puis peuplé de jeunes adultes issus du tri des adultes.
Tri/Collecte des oeufs : voir la description ci-avant de cette étape.
Création du bac pour les oeufs : Ce bac peut ensuite être peuplé avec la fraction oeufs issu de la collecte ci-avant, en prenant en compte le fait que la masse d'oeufs purs est de 55% pour adapter la densité souhaitée d'oeufs, ou avec des oeufs purs et propres issus de l'étape de séparation supplémentaire. Les oeufs vont éclore 6 à 10 jours après création du bac pour les oeufs afin de donner des larves.
Bac de pontes peuplé d'adultes : comme indiqué ci-avant un bac de ponte est créé puis peuplé d'adultes récupérés à l'issus de l'étape de tri et collecte des oeufs.

## Revendications

1. Milieu de ponte pour insectes comportant :
∘ au moins 80% en poids d'un substrat solide destiné à être consommé par les insectes sous forme de particules, au moins 85% en poids desdites particules ayant une taille de particules inférieure à 0,5 mm, ledit substrat solide ayant une humidité comprise entre 0 et 15%, et
∘ au moins 2% en poids d'un gel aqueux et optionnellement nutritif,
les pourcentages en poids étant donnés sur le poids total de milieu de ponte pour insectes.

2. Milieu de ponte pour insectes selon la revendication 1, dans lequel le substrat solide est un produit ou co-produit solide issu de la transformation des céréales, des oléogineux, des oléoprotéagineux et/ou des protéagineux.

3. Milieu de ponte pour insectes selon la revendication 1 ou 2, dans lequel le gel aqueux et optionnellement nutritif comporte :
∘ au moins 90% en poids d'une solution aqueuse,
∘ de 0,3 à 2% en poids d'un agent gélifiant, et
∘ de 0,1 à 5% en poids d'un agent conservateur,
les pourcentages en poids étant exprimés sur le poids total de gel.

4. Milieu de ponte pour insectes selon l'une quelconque des revendications 1 à 3, dans lequel le gel aqueux et optionnellement nutritif a une force de gel d'au moins 20 g/cm².

5. Bac de ponte comportant un contenant et, sur le fond dudit contenant, un milieu de ponte selon l'une quelconque des revendications 1 à 4.

6. Bac de ponte comportant un contenant et, sur un fond dudit contenant :
∘ de 0,12 à 7,5 g/cm² d'un substrat solide destiné à être consommé par les insectes sous forme de particules, au moins 85% en poids desdites particules ayant une taille de particules inférieure à 0,5 mm, ledit substrat solide ayant une humidité comprise entre 0 et 15%.

7. Bac de ponte selon la revendication 6, comportant en outre sur le fond dudit contenant :
∘ de 0,006 à 0,325 g/cm² ou 0,0016 à 0,095 g/cm²/j d'un gel aqueux et optionnellement nutritif.

8. Utilisation du milieu de ponte pour insectes selon l'une quelconque des revendications 1 à 4 ou d'un bac de ponte selon l'une quelconque des revendications 5 à 7, pour l'élevage de coléoptères et/ou lépidoptères.

9. Procédé d'obtention d'oeufs d'insectes comportant les étapes de :
- Obtention d'un bac de ponte par la fourniture d'un contenant et le remplissage dudit contenant avec :
∘ un substrat solide destiné à être consommé par les insectes sous forme de particules, au moins 85% en poids desdites particules ayant une taille de particules inférieure à 0,5 mm, ledit substrat solide ayant une humidité comprise entre 0 et 15%,
pour obtenir un bac de ponte,
- introduction d'insectes adultes dans le bac de ponte, et
une étape subséquente de collecte des oeufs d'insectes.

10. Procédé d'obtention d'oeufs d'insectes selon la revendication 9, dans lequel l'étape de remplissage du contenant par le substrat solide est effectuée par apport de 0,12 à 7,5 g/cm² dudit substrat solide dans le contenant.

11. Procédé d'obtention d'oeufs d'insectes selon la revendication 9 ou 10, dans lequel l'étape d'obtention d'un bac de ponte comporte en outre le remplissage dudit contenant avec :
∘ 0,0016 à 0,095 g/cm²/j d'un gel aqueux et optionnellement nutritif.

12. Procédé d'obtention d'oeufs d'insectes selon l'une quelconque des revendications 9 à 11, dans lequel l'étape d'introduction des insectes adultes est effectuée à une densité surfacique dans le bac comprise entre 0,01 à 1,0 g/cm².

13. Procédé d'obtention d'oeufs d'insectes selon l'une quelconque des revendications 9 à 12, dans laquelle l'étape subséquente de collecte des oeufs est effectuée au moyen d'une étape de tri automatisé.

14. Procédé d'obtention d'oeufs d'insectes selon l'une quelconque des revendications 9 à 13, dans lequel les insectes adultes sont des coléoptères et/ou des lépidoptères.

## Patentansprüche

1. Eilegemittel für Insekten umfassend:
- mindestens 80 % Gewichtsanteil eines festen Substrats zum Verzehr durch Insekten in Form von Partikeln, wobei mindestens 85 % Gewichtsanteil der Partikel eine Partikelgröße von weniger als 0,5 mm aufweisen und das feste Substrat eine Feuchtigkeit zwischen 0 und 15 % aufweist, und
- mindestens 2 % Gewichtsanteil eines wässrigen und optional nährenden Gels,
wobei sich die Prozentangaben in Gewichtsanteilen auf das Gesamtgewicht des Eilegemittel für Insekten beziehen.

2. Eilegemittel für Insekten nach Anspruch 1, wobei das feste Substrat ein festes Produkt oder Nebenprodukt aus der Verarbeitung von Getreide, Ölsaaten, Ölfrüchten und/ oder Eiweißpflanzen ist.

3. Eilegemittel für Insekten nach Anspruch 1 oder 2, wobei das wässrige und optional nährende Gel Folgendes umfasst:
- mindestens 90 % Gewichtsanteil einer wässrigen Lösung,
- von 0,3 bis 2 % Gewichtsanteil eines Geliermittels, und
- von 0,1 bis 5 % Gewichtsanteil eines Konservierungsmittels,
wobei sich die Prozentangaben in Gewichtsanteilen auf das Gesamtgewicht des Gels beziehen.

4. Eilegemittel für Insekten nach einem der Ansprüche 1 bis 3, wobei das wässrige und optional nährende Gel eine Gelstärke von mindestens 20g/cm² aufweist.

5. Eilegewanne umfassend einen Behälter und, auf dem Boden des Behälters ein Eilegemittel nach einem der Ansprüche 1 bis 4.

6. Eilegewanne umfassend einen Behälter und, auf einem Boden des Behälters:
- von 0,12 bis 7,5 g/cm² eines festen Substrats zum Verzehr durch Insekten in Form von Partikeln, wobei mindestens 85 % Gewichtsanteil der Partikel eine Partikelgröße von weniger als 0,5 mm aufweisen und das feste Substrat eine Feuchtigkeit zwischen 0 und 15 % aufweist.

7. Eilegewanne nach Anspruch 6, ferner auf dem Boden des Behälters umfassend:
- von 0,006 bis 0,325 g/cm² oder 0,0016 bis 0,095 g/cm²/d eines wässrigen und optional nährenden Gels.

8. Verwendung des Eilegemittels für Insekten nach einem der Ansprüche 1 bis 4 oder eines Eilegebehälters nach einem der Ansprüche 5 bis 7 zur Aufzucht von Käfern und/ oder Lepidopteren.

9. Verfahren zur Gewinnung von Insekteneiern, umfassend die folgenden Schritte:
- Gewinnung eines Eilegebehälters durch Bereitstellung eines Behälters und Befüllung des Behälters mit:
-- einem festen Substrat zum Verzehr durch Insekten in Form von Partikeln, wobei mindestens 85 % Gewichtsanteil der Partikel eine Partikelgröße von weniger als 0,5 mm aufweisen, wobei das feste Substrat eine Feuchtigkeit zwischen 0 und 15 % aufweist,
zur Gewinnung eines Eilegebehälters,
- Einführung von erwachsenen Insekten in den Eilegebehälter, und
ein anschließender Schritt des Sammelns von Insekteneiern.

10. Verfahren zur Gewinnung von Insekteneiern nach Anspruch 9, wobei der Schritt des Befüllens des Behälters mit dem festen Substrat durch Einbringen von 0,12 bis 7,5 g/cm² des festen Substrats in den Behälter durchgeführt wird.

11. Verfahren zur Gewinnung von Insekteneiern nach Anspruch 9 oder 10, wobei der Schritt zur Gewinnung eines Eilegebehälters ferner das Einbringen in den Behälter mit Folgendem umfasst:
- 0,0016 bis 0,095 g/cm²/d eines wässrigen und optional nährenden Gels.

12. Verfahren zur Gewinnung von Insekteneiern nach einem der Ansprüche 9 bis 11, wobei der Schritt des Einbringens der erwachsenen Insekten bei einer Flächendichte in der Wanne zwischen 0,01 und 1,0 g/cm² durchgeführt wird.

13. Verfahren zur Gewinnung von Insekteneiern nach einem der Ansprüche 9 bis 12, wobei der nachfolgende Schritt des Sammelns der Eier mithilfe eines automatisierten Sortierschrittes vorgenommen wird.

14. Verfahren zur Gewinnung von Insekteneiern nach einem der Ansprüche 9 bis 13, wobei es sich bei den erwachsenen Insekten um Käfer und/ oder Lepidopteren handelt.

## Claims

1. Egg-laying medium for insects comprising:
∘ at least 80% by weight of a solid substrate intended to be consumed by the insects in the form of particles, at least 85% by weight of said particles having a particle size smaller than 0.5 mm, said solid substrate having a moisture content comprised between 0 and 15%, and
∘ at least 2% by weight of an aqueous and optionally nutritional gel,
the percentages by weight being given in relation to the total weight of egg-laying medium for insects.

2. Egg-laying medium for insects according to claim 1, in which the solid substrate is a solid product or co-product originating from the conversion of cereals, oilseeds, protein-oil crops and/or protein crops.

3. Egg-laying medium for insects according to claim 1 or 2, in which the aqueous and optionally nutritional gel comprises:
∘ at least 90% by weight of an aqueous solution,
∘ 0.3 to 2% by weight of a gelling agent, and
∘ 0.1 to 5% by weight of a preservative,
the percentages by weight being expressed in relation to the total weight of gel.

4. Egg-laying medium for insects according to any one of claims 1 to 3, in which the aqueous and optionally nutritional gel has a gel strength of at least 20 g/cm².

5. Laying tray comprising a container and, on the bottom of said container, an egg-laying medium according to any one of claims 1 to 4.

6. Laying tray comprising a container and, on the bottom of said container:
∘ 0.12 to 7.5 g/cm² of a solid substrate intended to be consumed by the insects in the form of particles, at least 85% by weight of said particles having a particle size smaller than 0.5 mm, said solid substrate having a moisture content comprised between 0 and 15%.

7. Laying tray according to claim 6, moreover comprising, on the bottom of said container:
∘ 0.006 to 0.325 g/cm² or 0.0016 to 0.095 g/cm²/d of an aqueous and optionally nutritional gel.

8. Use of the egg-laying medium for insects according to any one of claims 1 to 4 or a laying tray according to any one of claims 5 to 7, for breeding coleopterans and/or lepidopterans.

9. Method for obtaining insect eggs, comprising the steps of:
- obtaining a laying tray by providing a container and filling said container with:
∘ a solid substrate intended to be consumed by the insects in the form of particles, at least 85% by weight of said particles having a particle size smaller than 0.5 mm, said solid substrate having a moisture content comprised between 0 and 15%,
in order to obtain a laying tray,
- introducing adult insects into the laying tray, and
a subsequent step of collecting the insect eggs.

10. Method for obtaining insect eggs according to claim 9, in which the step of filling the container with the solid substrate is effected by supplying 0.12 to 7.5 g/cm² of said solid substrate into the container.

11. Method for obtaining insect eggs according to claim 9 or 10, in which the step of obtaining a laying tray moreover comprises filling said container with:
∘ 0.0016 to 0.095 g/cm²/d of an aqueous and optionally nutritional gel.

12. Method for obtaining insect eggs according to any one of claims 9 to 11, in which the step of introducing the adult insects is effected in an areal density in the laying tray comprised between 0.01 and 1.0 g/cm².

13. Method for obtaining insect eggs according to any one of claims 9 to 12, in which the subsequent step of collecting the eggs is effected by means of an automated sorting step.

14. Method for obtaining insect eggs according to any one of claims 9 to 13, in which the adult insects are coleopterans and/or lepidopterans.
